# EUROPÄISCHE PATENTANMELDUNG

(11) **EP 1 293 496 A2**
(43) Veröffentlichungstag der Anmeldung: **19.03.2003**
(21) Anmeldenummer: 02020470.7
(22) Anmeldetag: 12.09.2002
(51) Int. Cl.: C07C 209/26

(54) **Verfahren zur Herstellung von Aminen**

(30) Priorität: 13.09.2001 DE 10145119
(71) Anmelder: BASF AKTIENGESELLSCHAFT, 67056 Ludwigshafen (DE)
(72) Erfinder: Schäfer, Martin, Dr., 67269 Grünstadt (DE); Böttcher, Arndt, Dr., 67227 Frankenthal (DE); Kramer, Andreas, Dr., 67098 Bad Drükheim (DE); Höhn, Arthur, Dr., 67281 Kirchheim (DE); Liang, Shelue, Dr., 67071 Ludwigshafen (DE); Funke, Frank, Dr., 68159 Mannheim (DE)
(74) Vertreter: Isenbruck, Günter, Dr.

(57) **Zusammenfassung**

Die Herstellung von Aminen der allgemeinen Formel ( I )

R¹R²CH-NR³R⁴ (I)

in der R¹, R², R³ und R⁴ unabhängig voneinander Wasserstoff, geradkettiges oder verzweigtes C₁-C₁₂-Alkyl, C₃-C₁₂-Cycloalkyl, C₆-C₁₀-Aryl oder C₇-C₁₁-Aralkyl bedeuten, erfolgt durch katalytische, hydrierende Aminierung von Carbonylverbindungen der allgemeinen Formel (II) und/oder Alkohole der allgemeinen Formel (III)

R¹-C(=O)-R² (II)

R¹-CH(OH)-R² (III)

enthaltenden Gemischen, die ferner mindestens 50 ppm, bezogen auf die Gemische, an Halogen enthalten,
mit Stickstoffverbindungen der allgemeinen Formel (IV)

H N R³ R⁴ (IV)

mit den vorstehend genannten Bedeutungen für R¹ bis R⁴,
in Gegenwart von Co- und/oder Ni-haltigen Katalysatoren, dadurch gekennzeichnet, dass die hydrierende Aminierung zusätzlich in Gegenwart von festen sauren Cokatalysatoren durchgeführt wird.

## Beschreibung

Die Erfindung betrifft ein Verfahren zur Herstellung von Aminen durch katalytische hydrierende Aminierung von Carbonylverbindungen oder Alkoholen, die halogenhaltige Verunreinigungen enthalten.

Die hydrierende Aminierung von Carbonylverbindungen mit Ammoniak oder primären oder sekundären Aminen ist ein Standardverfahren zur Herstellung von Aminen. Üblicherweise wird die Umsetzung in Gegenwart von metallischen Katalysatoren durchgeführt, wobei beispielsweise Raney-Ni, Raney-Co, Pt/Aktivkohle, Pd/Aktivkohle, Pd/BaSO₄, Rh/Al₂O₃ als Katalysatoren zum Einsatz kommen.

Enthält die zu aminierende Carbonylverbindung herstellbedingt signifikante Mengen Chlor (zum Beispiel > 50 ppm), führt dies zu Problemen bei der hydrierenden Aminierung. So resultieren zum Beispiel geringere Selektivitäten und Raum-Zeit-Ausbeuten sowie eine vorzeitige Desaktivierung oder Zerstörung des Katalysators. In vielen Fällen kann nur ein Teilumsatz erzielt werden, was die Aufarbeitung erschwert. Insbesondere Festbetthydrierungen sind in Gegenwart von Halogenen bzw. halogenhaltigen Verunreinigungen nicht wirtschaftlich durchführbar aufgrund des Zerfallens des Katalysators.

In diesen Fällen muss die Carbonylverbindung vor der Aminierung in aufwendiger Weise aufgereinigt werden. In vielen Fällen ist dies nicht mit wirtschaftlich vertretbarem Aufwand möglich.

Die prioritätsältere, nicht vorveröffentlichte DE-A-100 53 380 betrifft ein Verfahren zur Herstellung von Aminen durch katalytische hydrierende Aminierung von Carbonylverbindungen oder Alkoholen, die mindestens einen durch Halogen substituierten aromatischen Kern aufweisen, der bei der hydrierenden Aminierung nicht verändert wird. Die Hydrierung wird in Gegenwart von Co- und/oder Ni-haltigen Katalysatoren und festen sauren Cokatalysatoren und in Abwesenheit von organischen Schwefelverbindungen durchgeführt.

Aufgabe der vorliegenden Erfindung ist die Bereitstellung eines Verfahrens zur einfachen und kostengünstigen Herstellung von Aminen durch hydrierende Aminierung entsprechender Carbonylverbindungen oder Alkohole, die einen signifikanten Gehalt an Halogen (mindestens 50 ppm, insbesondere organisch gebunden) aufweisen, und das mit hohen Raum-Zeit-Ausbeuten bei niedrigem Katalysatorverbrauch zu den gewünschten Produkten führt.

Die Aufgabe wird erfindungsgemäß gelöst durch ein Verfahren zur Herstellung von Aminen der allgemeinen Formel ( I )

R¹ R² CH-N R³ R⁴ ( I )

in der R¹, R², R³ und R⁴ unabhängig voneinander Wasserstoff, geradkettiges oder verzweigtes C₁-C₁₂-Alkyl, C₃-C₁₂-Cycloalkyl, C₆-C₁₀-Aryl oder C₇-C₁₁-Aralkyl bedeuten, wobei mindestens einer der Reste R¹ und R² Aryl oder Aralkyl darstellt,
durch katalytische, hydrierende Aminierung von Carbonylverbindungen der allgemeinen Formel ( II ) oder Alkohole der allgemeinen Formel ( III )

R¹-C(=O)-R² ( II )

R¹-CH(OH)-R² ( III )

enthaltenden Gemischen, die ferner mindestens 50 ppm, bezogen auf die Gemische, an Halogen enthalten,
mit Stickstoffverbindungen der allgemeinen Formel ( IV )

H N R³R⁴ ( IV )

mit den vorstehend genannten Bedeutungen für R¹ bis R⁴
in Gegenwart von Co- und/oder Ni-haltigen Katalysatoren, wobei die hydrierende Aminierung zusätzlich in Gegenwart von festen sauren Cokatalysatoren und gegebenenfalls in Abwesenheit von organischen Schwefelverbindungen durchgeführt wird.

Es wurde erfindungsgemäß gefunden, dass bei der hydrierenden Aminierung von Ketonen, Aldehyden oder Alkoholen, die durch signifikante Mengen Halogen verunreinigt sind, sehr gute Amin-Ausbeuten erzielt werden können, wenn man bei der Umsetzung die Cound/oder Ni-haltigen Katalysatoren in Kombination mit festen sauren Cokatalysatoren einsetzt.

Die Co- und/oder Ni-haltigen Katalysatoren sind vorzugsweise ausgewählt aus Raney-Co, Raney-Ni, Raney-Co-Ni, Raney-Ni-Fe, Raney-Ni-Fe-Co, Ni-Co-, Ni/Co-Mischoxiden oder Gemischen davon, die auch auf anorganischen Trägern vorliegen können.

Die Co- und/oder Ni-Katalysatoren können zudem zusätzlich 0 bis 80 Gew.-% Cu und 0 bis 10 Gew.-%, vorzugsweise 0 bis 5 Gew.-% weitere Metalle enthalten, berechnet als Metall und bezogen auf das Gesamtgewicht des Katalysators.

Im erfindungsgemäßen Verfahren werden die Co- und/oder Ni-haltigen Katalysatoren vorzugsweise in Form von elementarem Co- und/oder Ni-Schwamm, Raney-Cobalt oder Raney-Nickel, Co oder Ni bzw. Co-Oxid oder Ni-Oxid auf Trägern (Trägerkatalysatoren) verwendet. Sie können auch in beliebigen Gewichtsverhältnissen vermischt eingesetzt werden. Träger dafür sind z.B. Al₂O₃, SiO₂, TiO₂, ZrO₂, Aktivkohle und andere dem Fachmann bekannte Katalysatorträger.

Unter den Raney-Katalysatoren werden bevorzugt Raney-Katalysatoren wie Raney-Cobalt, Raney-Cobalt-Nickel, Raney-Cobalt-Nickel-Eisen, Raney-Cobalt-Nickel-Eisen-Chrom oder Raney-Cobalt oder Raney-Nickel mit Dotierungen anderer Übergangsmetalle in wasserfreier oder auch wasserfeuchter oder lösungsmittelfeuchter Form eingesetzt. Besonders bevorzugt wird Raney-Cobalt, das jeweils 0 oder 0,1 bis 10; vorzugsweise bis 5 Gew.-% Al, Ni, Fe, Cr enthält, verwendet. Der Co- oder Ni-haltige Katalysator wird in einer Menge von 0,01 bis 20 Gew.-%, bevorzugt 0,1 bis 10 Gew.-% und besonders bevorzugt 0,3 bis 5 Gew.-%, bezogen auf die zu aminierende Carbonylverbindung, eingesetzt.

Es kann erfindungsgemäß auch vorzugsweise ein Ni- oder Co-Ni-Katalysator eingesetzt werden, der auf ZrO₂ geträgert ist und 0 bis 50 Gew.-% CuO, berechnet als CuO und bezogen auf das Gesamtgewicht der Katalysators, enthält.

Die festen sauren Cokatalysatoren sind erfindungsgemäß vorzugsweise ausgewählt aus Metalloxiden oder Metallmischoxiden, Zeolithen, Metall- oder Ammoniumsalzen von Mineralsäuren oder organischen Säuren, sauren Ionentauschern oder Gemischen davon.

Als feste saure Kontakte im erfindungsgemäßen Verfahren werden bevorzugt Metalloxide, wie Oxide oder Mischoxide der Elemente Zr, Ti, Cr, Mo, W, Mn, Fe, B, Al, Si, Zeolithe natürlicher und synthetischer Herkunft, Metall- oder Ammoniumsalze starker Mineralsäuren wie Salzsäure, Schwefelsäure, Salpetersäure, Phosphorsäure und starker organischen Säuren wie Ameisensäure, Essigsäure, Propionsäure und Sulfonsäure, saure Ionentauschern wie Nafion usw. verwendet. Insbesondere wird ZrO₂ eingesetzt. Die Einsatzmengen der sauren Kontakte liegen vorzugsweise zwischen 0,1 und 20 Gew.-%, bevorzugt zwischen 1-10 Gew.-%, bezogen auf die zu aminierende Carbonylverbindung.

Beim Einsatz von Co- oder Ni-haltigen Trägerkatalysatoren können die Trägermaterialien wie Al₂O₃, SiO₂, TiO₂, ZrO₂ usw. als saure Kontakte fungieren. In diesem Fall ist die Zugabe eines zusätzlichen sauren Kontakts nicht nötig. Kommen die Ammoniumsalze als saure Kontakte zum Einsatz, so kann anstatt der Ammoniumsalze die herkömmliche Säure direkt eingesetzt werden. Die Ammoniumsalze bilden sich dann durch die Reaktion der Säure mit Ammoniak oder Aminen.

Im erfindungsgemäßen Verfahren werden als saure Kontakte vorzugsweise Metalloxide oder deren Mischungen eingesetzt. Besonders bevorzugt ist ZrO₂.

Die hydrierende Aminierung kann in Suspensions- oder Festbettfahrweise durchgeführt werden.

Die erfindungsgemäß eingesetzten Carbonylverbindungen oder Alkohole können weitgehend frei gewählt werden.

Besonders bevorzugt handelt es sich bei den Arylresten um Phenylreste und bei den Aralkylresten um Benzylreste. Die Alkylreste sind vorzugsweise C₁₋₆-Alkylreste, die Cycloalkylreste C₃₋₆-Cycloalkylreste. Besonders bevorzugt ist in den Verbindungen der allgemeinen Formel ( II ) und ( III ) keiner der Reste Wasserstoff. Insbesondere ist die Carbonylverbindung Pinakolon oder ein Derivat davon.

Die erfindungsgemäß eingesetzten zu aminierenden Carbonylverbindungen oder Alkohole enthalten mindestens 50 ppm, besonders bevorzugt 50 ppm bis 10 Gew.-%, insbesondere 100 ppm bis 10 Gew.-% an Halogen. Die Mengenangaben beziehen sich dabei auf ein Gemisch aus den Carbonylverbindungen oder Alkoholen und dem Halogen bzw. den halogenhaltigen Verbindungen. Der Ausdruck "Halogen" und die Mengenangabe beziehen sich auf im Gemisch vorliegende Halogenatome, die in chemischer Form gebunden oder als Ionen oder Salze vorliegen können. Insbesondere handelt es sich um organisch gebundenes Halogen, das heißt organische Verbindungen, die Halogenatome, insbesondere Chloratome, chemisch gebunden enthalten. Die Mengenangaben beziehen sich jedoch auf das Halogen selbst unabhängig von der Art und Größe des organischen Restes. In der Regel liegt das Halogen als ionische Verunreinigung oder insbesondere gebunden an organische Verunreinigungen in der eingesetzten Carbonylverbindung vor.

Bei dem erfindungsgemäß eingesetzten Gemisch handelt es sich damit insbesondere um mit organischen Chlorverbindungen verunreinigtes Pinakolon oder dessen Derivate.

Bei der Stickstoffverbindung der Formel (IV) handelt es sich vorzugsweise um Ammoniak oder primäre oder sekundäre aliphatische Amine. Letztere weisen vorzugsweise C₁₋₆-Alkylreste auf. Besonders bevorzugt wird Ammoniak als Stickstoffverbindung eingesetzt.

Die Aminierung im erfindungsgemäßen Verfahren kann in Lösungsmitteln, beispielsweise Alkoholen wie Methanol, Ethanol, Propanol, Butanol, aliphatischen oder aromatischen Kohlenwasserstoffen wie Toluol, Xylol, Cyclohexan, Isooctan, Ether wie Tetrahydrofuran, Dioxan, Methyl-tert-butylether durchgeführt werden. In einer bevorzugten Durchführung wird jedoch kein zusätzliches Lösungsmittel verwendet, sondern das im Überschuss eingesetzte Aminierungsmittel (Ammoniak oder Amine) fungiert gleichzeitig als Lösungsmittel. Das Molverhältnis von Ammoniak oder Amin zu Carbonylverbindung liegt vorzugsweise bei größer 1, für schnelle Reaktionen vorzugsweise bei größer 2.

Die Aminierung wird vorzugsweise bei einer Temperatur von 10 bis 250°C, bevorzugt von 40 bis 150°C und bei einem Druck von 10 bis 200 bar, bevorzugt von 30 bis 130 bar durchgeführt.

Das erfindungsgemäße Verfahren kann im Festbett durchgeführt werden, aber auch z.B. in einem Autoklaven in Suspension, wie es nachstehend beschrieben ist. Die Co- oder Ni-haltigen und die sauren Cokatalysatoren sowie die Carbonylverbindung werden unter Schutzgas in einer beliebigen Reihfolge in den Reaktor eingefüllt. Bei Raumtemperatur werden Ammoniak oder Amine unter Rühren zudosiert. Die Mischung wird auf Reaktionstemperatur aufgeheizt, und anschließend wird Wasserstoff auf Reaktionsdruck aufgepresst. Nach Beendigung der Wasserstoff-Aufnahme wird der Reaktor auf Raumtemperatur abgekühlt, entspannt und entleert. Die Katalysatoren werden abfiltriert und für den nächsten Reaktionszyklus wieder eingesetzt. Die Aufarbeitung des Reaktionsproduktes erfolgt dann in dem Fachmann bekannter Weise.

Das Verfahren kann sowohl diskontinuierlich, beispielsweise in einem Autoklaven, wie auch kontinuierlich, beispielsweise in einem Druckrohr mit angeschlossenem Abscheider und Druckentspannung, durchgeführt werden.

Durch Verwendung von Co- und/oder Ni-haltigen Katalysatoren in Kombination mit sauren Cokatalysatoren wird die hydrierende Aminierung von halogenhaltigen Edukten ermöglicht; es können hohe Raum-Zeit-Ausbeuten bei nur geringen Katalysatorverlusten erzielt werden. Die hohen Amin-Selektivitäten und Ausbeuten im erfindungsgemäßen Verfahren, kombiniert mit einfacher Durchführung der Synthese ermöglichen eine einfache Aufarbeitung und damit insgesamt ein wirtschaftliches Herstellungsverfahren.

Das erfindungsgemäße Verfahren wird anhand folgender Beispiele mit Pinakolon (3,3-Dimethylbutanon) als stellvertretende Carbonylverbindung näher erläutert.

### Beispiele:

Das in den Beispielen eingesetzte Pinakolon hatte eine Reinheit von > 98,4 % (lt. GC), der Gehalt an organisch gebundenem Halogen betrug 280 ppm. Die dabei verwendeten Katalysatoren sind ebenfalls Handelsware mit folgender Zusammensetzung:
- Ra-Ni:: Raney-Nickel, Suspensionskatalysator
- Al₂O₃:: Pulver
- ZrO₂:: Pulver

Die Aminierungsversuche wurden nach der unten stehenden allgemeinen Arbeitsvorschrift durchgeführt und sind nicht optimiert. Höhere Amin-Selektivitäten und -Ausbeuten sind daher möglicherweise erreichbar.

### Allgemeine Arbeitsvorschrift:

In einem mit Stickstoff gespülten und gefüllten 2,5 l Autoklaven mit einem Scheibenrührer und zwei Wellenbrechern werden die Katalysatoren, Pinakolon (Cl-Gehalt (org. gebunden): 280 ppm) und als Lösungsmittel Methanol vorgelegt. Bei Raumtemperatur werden unter Rühren (500 U/min) zunächst Ammoniak und dann Wasserstoff auf 20 bar aufgepresst. Die Mischung wird auf Reaktionstemperatur aufgeheizt, mit Wasserstoff auf Reaktionsdruck nachgepresst und anschließend unter konstantem Wasserstoff-Druck gerührt. Nach Beendigung der Wasserstoff-Aufnahme wird der Autoklav abgekühlt, entspannt, ausgegast und entleert. Die Zusammensetzungen der Reaktionsausträge werden gaschromatographisch bestimmt. Die Versuchsergebnisse sind in Tabelle 1 zusammengestellt.

**Tabelle 1:**

| Ergebnisse der Aminierung von Pinakolon (3,3-Dimethylbutanon) | | | | | | | | |
|---|---|---|---|---|---|---|---|---|
| Beispiel | Katalysatoren (g) | Pinakolon/NH₃ /MeOH [g] | T/P [°C/bar] | Ums. [%] | Amin [%] | Alk.^{a}[%] | SB^{b}[%] | Zeit/h |
| 1 | Ra-Ni (15) / ZrO₂(25) | 500 / 255 / 250 | 150 / 180 | 97 | 94 | 0,6 | 1,2 | 30 |
| V2 | Ra-Ni (15) | 500 / 255 / 250 | 150 / 180 | 72 | 59 | 0,5 | 10,9 | 30 |
| 3 | Ra-Ni (6) / ZrO₂ (15) | 300 / 170 / 150 | 110 / 100 | 94 | 86 | 2 | 5 | 30 |

| | | | | | | | | |
|---|---|---|---|---|---|---|---|---|
| V2 = Vergleichsbeispiel | | | | | | | | |
| a. Alk. = 3,3-Dimethylbutanol, | | | | | | | | |
| b. SB = Schiff sche Base aus 3,3-Dimethylbutanon und 3,3-Dimethylbutylamin. | | | | | | | | |

Aus den vorstehenden Versuchsergebnissen geht hervor, dass unter Verwendung der sauren Cokatalysatoren wesentlich höhere Umsätze und Produktselektivitäten erreicht werden konnten.

## Patentansprüche

1. Verfahren zur Herstellung von Aminen der allgemeinen Formel ( I )
R¹R²CH-NR³R⁴ ( I )
in der R¹, R², R³ und R⁴ unabhängig voneinander Wasserstoff, geradkettiges oder verzweigtes C₁-C₁₂-Alkyl, C₃-C₁₂-Cycloalkyl, C₆-C₁₀-Aryl oder C₇-C₁₁-Aralkyl bedeuten,
durch katalytische, hydrierende Aminierung von Carbonylverbindungen der allgemeinen Formel ( II ) und/oder Alkohole der allgemeinen Formel ( III )
R¹-C(=O)-R² ( II )
R¹-CH(OH)-R² ( III )
enthaltenden Gemischen, die ferner mindestens 50 ppm, bezogen auf die Gemische, an Halogen enthalten,
mit Stickstoffverbindungen der allgemeinen Formel ( IV )
HNR³R⁴ ( IV )
mit den vorstehend genannten Bedeutungen für R¹ bis R⁴,
in Gegenwart von Co- und/oder Ni-haltigen Katalysatoren, **dadurch gekennzeichnet, dass** die hydrierende Aminierung zusätzlich in Gegenwart von festen sauren Cokatalysatoren durchgeführt wird.

2. Verfahren nach Anspruch 1, **dadurch gekennzeichnet, dass** die festen sauren Cokatalysatoren ausgewählt sind aus Metalloxiden oder Metallmischoxiden, Zeolithen, Metall- oder Ammoniumsalzen von Mineralsäuren oder organischen Säuren, sauren Ionentauschern oder Gemischen davon.

3. Verfahren nach Anspruch 1 oder 2, **dadurch gekennzeichnet, dass** die Co- und/oder Ni-haltigen Katalysatoren ausgewählt sind aus Raney-Co, Raney-Ni, Raney-Ni-Fe, Raney-Ni-Fe-Co, Ni-, Co-, Ni/Co-Mischoxiden oder Gemischen davon, die auch auf anorganischen Trägern vorliegen können.

4. Verfahren nach einem der Ansprüche 1 bis 3, **dadurch gekennzeichnet, dass** die Cound/oder Ni-haltigen Katalysatoren zusätzlich 0 bis 80 Gew.-% Cu und 0 bis 10 Gew.-% weitere Metalle enthalten, berechnet als Metall und bezogen auf das Gesamtgewicht des Katalysators.

5. Verfahren nach Anspruch 4, **dadurch gekennzeichnet, dass** ein Raney-Ni-Katalysator eingesetzt wird, der zusätzlich jeweils 0 bis 10 Gew.-% Al, Co, Cr und/oder Fe enthält, berechnet als Metall und bezogen auf das Gesamtgewicht des Katalysators.

6. Verfahren nach Anspruch 4, **dadurch gekennzeichnet, dass** ein Ni-Katalysator eingesetzt wird, der auf ZrO₂ geträgert ist und 0 bis 50 Gew.-% CuO enthält, berechnet als CuO und bezogen auf das Gesamtgewicht des Katalysators.

7. Verfahren nach einem der Ansprüche 1 bis 6, **dadurch gekennzeichnet, dass** als feste saure Cokatalysatoren Oxide und Mischoxide der Elemente Zr, Ti, Cr, Mo, W, Mn, Fe, B, Al, Si oder deren Gemische, Zeolithe, Metall- oder Ammoniumsalze von Salzsäure, Schwefelsäure, Salpetersäure, Phosphorsäure oder Ameisensäure, Essigsäure, Propionsäure oder Sulfonsäure oder saure Ionentauscher verwendet werden.

8. Verfahren nach Anspruch 7, **dadurch gekennzeichnet, dass** als fester saurer Cokatalysator ZrO₂ verwendet wird.

9. Verfahren nach einem der Ansprüche 1 bis 8, **dadurch gekennzeichnet, dass** die Katalysatoren und Cokatalysatoren in Suspension eingesetzt werden.

10. Verfahren nach einem der Ansprüche 1 bis 9, **dadurch gekennzeichnet, dass** Pinakolon als Carbonylverbindung und Ammoniak als Stickstoffverbindung verwendet werden.
